# EUROPÄISCHE PATENTSCHRIFT

(11) **EP 1 547 689 B1**
(45) Veröffentlichungstag und Bekanntmachung des Hinweises auf die Patenterteilung: **27.10.2010**
(21) Anmeldenummer: 04027940.8
(22) Anmeldetag: 25.11.2004
(51) Int. Cl.: B01L 3/00, G01N 33/49, G01N 33/86

(54) **Kartusche zur Funktionskontrolle einer Vorrichtung für die Untersuchung der Blutplättchenfunktion, Verfahren zur Funktionskontrolle und Verwendung einer Testflüssigkeit**
Cartridge for use in the control of the functionality of a device for the analysis of blood platelet functionality, process for the control of functionality, and use of a test liquid
Cartouche servant au contrôle de la fonctionnalité d'un appareil pour l'analyse de la fonctionnalité des plaquettes sanguines, procédé de contrôle d'une fonctionnalité, ainsi que l'utilisation d'un liquide d'essai.

(30) Priorität: 23.12.2003 DE 10360814
(43) Veröffentlichungstag der Anmeldung: 29.06.2005
(73) Patentinhaber: Siemens Healthcare Diagnostics Products GmbH, 35041 Marburg (DE)
(72) Erfinder: De Haan, Jacob Dr., 35315 Homberg/Ohm (DE)

(56) Entgegenhaltungen:
- WO-A-96/00899
- US-A- 4 858 127
- US-A- 5 051 239
- US-A- 5 602 037
- US-A- 5 925 319

## Beschreibung

Die Erfindung betrifft eine Kartusche zur Funktionskontrolle einer Vorrichtung für die Untersuchung der Blutplättchenfunktion, mit einem Gehäuse, das eine Testkammer und eine Haltekammer umschließt und ein Verfahren zur Funktionskontrolle einer derartigen Vorrichtung sowie die Verwendung einer Testflüssigkeit in der Vorrichtung.

In einer Vorrichtung für die automatisierte Untersuchung der Blutplättchenfunktion werden Testkartuschen eingesetzt, die bioaktive poröse Trennelemente enthalten. Mit der Vorrichtung werden Untersuchungen oder Tests des Blutgerinnungsprozesses anhand der Blutplättchenfunktion durchgeführt, wobei einige oder alle Schritte einer Untersuchung automatisch ablaufen.

Die Hämostase oder Blutstillung beinhaltet das Zusammenspiel von zwei biochemischen Systemen, die durch verschiedene Proteinfaktoren und zelluläre Komponenten, z. B. Blutplättchen, gesteuert werden. Die Vorgänge, durch die Blut gerinnt, beinhalten nach derzeitigem Verständnis eine mehrstufige Kaskade von Aktivierungen der Proteinfaktoren, die in der Fibrinbildung gipfeln. Verschiedene. Tests wurden entwickelt, um die einzelnen Stufen dieser Kaskade zu testen und so bestimmen zu können, ob das Blut eines Patienten einwandfrei gerinnen kann oder ob eine Gerinnungsstörurig mit einem Mangel an einem oder mehreren der zur Blutgerinnung notwendigen Faktoren vorliegt. Es ist bekannt, dass der Zustand der Blutplättchen oder die Blutplättchenfunktion einen Hinweis auf die Fähigkeit des Blutes zur einwandfreien Gerinnung gibt.

Der Test zum Untersuchen der Plättchenfunktiön oder der primären Hämostase an menschlichem Vollblut ist als Blutungszeittest bekannt. Der Blutungszeittest, der mehrere Dekaden angewandt wurde, beinhaltet einen Einschnitt am Unterarm des Patienten. Zur Vermeidung eines Einschnitts wurde daher ein weiterer Test entwickelt, der wesentlich genauer die Blutplättchendiagnose erstellen kann.

Die US-Patente 4 604 804, 4 780 417 und 5 051 239 offenbaren ein Assaysystem, das verwendet werden kann, um einen in-vitro-Test mit Blut durchzuführen, welcher genau und reproduzierbar mit dem oben beschriebenen in-vivo-Blutungszeittest korreliert werden kann. Das Thrombostat™ 4000 Gerät ist ein solches System. Die Blutplättchenfunktion wird in diesem System bewertet, indem antikoagulierte Vollblutproben mit einem ständigen Unterdruck durch eine kleine Öffnung gesaugt werden, die sich in der Mitte einer Trennwand befindet, welche nichtporös oder porös sein kann. Bei Systemen, bei denen die Trennwand porös ist, wird sie vor Beginn des Assays mit einem Aktivator benetzt, der die Koagulation der Blutplättchen aktiviert. An der Öffnung bildet sich ein Thrombocytenpfropf, und es wird die zum Stoppen des Blutflusses erforderliche Zeit gemessen. Diese Zeit wird dann mit der Blutplättchenfunktion korreliert.

Die mit dem Thrombostat™ 4000 verwendete Vorrichtung besteht aus drei getrennten Teilen: einer Reagenz-/Testkammer, einer Kapillare und einem Probenapf. Eine poröse Trennwand, die Collagen enthält, befindet sich in der Reagenz-/Testkammer. Die Reagenz-/Testkammer muss in einer hermetischen Verpackung, von der Kapillare und dem Probenapf getrennt, aufbewahrt werden, um die Stabilität des Collagens während der angegebenen Lagerungszeit aufrecht zu erhalten. Die Kapillare und die Reagenz-/Testkammer müssen zu Beginn jedes durchgeführten Tests von der Bedienperson manuell zusammengesetzt werden. Weiterhin muss die zu testende Probe in den Probenapf pipettiert und inkubiert werden, bevor der Probenapf mit der Kapillare und der Reagenz-/Testkammer zusammengesetzt werden kann. Außerdem wird die Zeit für den Inkubationsschritt von der Bedienperson manuell bestimmt. Der getrennte Inkubationsschritt erfordert eine zusätzliche Handhabung nach der Inkubationszeit, wenn die Bedienperson die zusammengesetzte Kapillare und Reagenz-/Testkammer manuell in den Probenapf einsetzt und die Testsequenz initiiert. Am Ende des Tests wird die teure Kapillare wieder verwendet und muss daher zeitaufwändig gereinigt werden.

Zur Vermeidung dieser Nachteile ist aus der EP 0 716 745 B1 eine Kartusche bekannt, bei der der Anwender während des Testzyklus nicht mehr einzugreifen braucht. Diese Testkartusche benötigt keine komplizierten Probenhandhabungsmechanismen, sie macht eine getrennte externe hermetische Verpackung für die Reagenz-/Testkammern während Transport und Lagerung überflüssig, und sie ist zur einmaligen Verwendung vorgesehen. Die Testkartusche ist im allgemeinen für Assaysysteme geeignet, bei denen bestimmte Komponenten/Reagenzien getrennt gehalten oder erst zum geeigneten Zeitpunkt miteinander kombiniert werden.

Die komplexen Vorgänge in der Primär-Hämostase führen über Thrombocytenadhäsion und -aggregation zur Pfropfbildung. Bekannte Geräte messen die Zeit, die unter standardisierten Bedingungen hierfür notwendig ist. Das Ergebnis der Zeitmessung ist die sogenannten Verschlusszeit, die in Sekunden angegeben wird. Sie ist ein Maß für die Plättchen-Hämostase-Kapazität.

Bei der Untersuchung werden plasmatische und zelluläre Komponenten der Primär-Hämostase erfasst. Dies geschieht durch in-vitro-Simulation der physiologischen Bedingungen, die zu Adhäsion, Aktivierung und Aggregation der Thrombocyten führen.

Bei den Vorrichtungen für die Plättchenfunktionsdiagnostik werden beim Durchfluss einer gepufferten Natriumcitrat-Vollblutprobe durch eine mit Collagen (Col) und einem weiteren Aktivator wie Epinephrin (Epi) oder Adenosin-5'-diphosphat (ADP) beschichtete Membranöffnung die Verhältnisse simuliert, die in einem Blutgefäß herrschen. Die Plättchen reagieren in Anwesenheit der plasmatischen Komponenten, beispielsweise des v. Willebrand-Faktors unter Druck- und Scherkraftverhältnissen, die denen eines kleinen verletzten Blutgefäßes entsprechen. Durch Adhäsion und Aggregation der Thrombocyten kommt es zum Verschluss der Membranöffnung. Die Zeit gemessen vom Beginn der Messung bis zum Verschluss der Membranöffnung ist die schon erwähnte Verschlusszeit. Die Entscheidungsgrenzen in klinischen Studien ergeben sich unter Berücksichtigung der sich überlappenden Verschlusszeiten normaler und anomaler Populationen beim PFA- 100® -System anhand folgender Referenzbereiche:

| Messzelle der Vorrichtung | 3,8 % gepuffertes Citratblut Referenzbereich (s) | 3,2 % gepuffertes Citratblut Referenzbereich (s) |
|---|---|---|
| Collagen /Epinephrin | 94 - 193 | 82 - 150 |
| Collagen /ADP | 71 - 118 | 62 - 100 |

Mit den bekannten Vorrichtungen für die Plättchenfunktionsdiagnostik steht für Risikopatienten mit angeborener Thrombocyten-Funktionsstörung somit eine einfache Screening-Möglichkeit zur Verfügung. Die Messzellen bzw. Testkartuschen dieser Vorrichtungen erlauben die Unterscheidung zwischen normaler und anomaler Plättchenfunktion (Col/Epi) und die Erkennung einer Acetylsalicylsäure induzierten Störung (Col/ADP). Durch die Plättchenfunktionsdiagnostik können sehr viele Patienten mit angeborenen Thrombocyten-Funktionsstörungen diagnostiziert werden, ohne dass eine weitere Spezialdiagnostik wie z. B. die Aggregometrie benötigt wird. Bei Einnahme von Acetylsalicylsäure werden im Serum sehr rasch ansteigende und wieder abklingende Medikamentenkonzentrationen gemessen. Die Folge ist ein Ansteigen der Col/Epi-Verschlusszeiten, die über Tage verlängert bleiben. Individuell sind bei Patienten unterschiedlich starke Verlängerungen der Verschlusszeiten festgestellt worden.

In den EP- 0 716 744 B1 und 0 716 745 B1 sowie in US-A-5602037 und WO96/00899A sind Vorrichtungen für die Blutplättchendiagnostik, in denen Messzellen bzw. Testkartuschen eingesetzt sind und die Assays zum Testen der Blutplättchenfunktion durchführen, ausführlich beschrieben. Die in den US-Patenteri 4 604 804, 4 780 418 und 5 051 239 beschriebenen Assays zum Testen der Plättchenfunktion umfassen einen Inkubationsschritt in der Vorrichtung, während dem die zu analysierende Blutprobe und die Komponenten des Assays auf eine bestimmte Temperatur erhitzt werden, wobei während dieses Inkubationsschritts die Probe und die Assaykomponenten getrennt gehalten werden. Nach dem Inkubationsschritt gelangt ein Kapillarrohr mit dem Blut in Kontakt, wobei ein Unterdruck in der Testkammer erzeugt wird, der bewirkt, dass Blut durch das Kapillarröhr angesaugt wird. Der Unterdruck bewirkt, dass die Blutprobe aus einer Haltekammer durch das Kapillarrohr hindurch in eine Aufnahmekammer und durch eine Öffnung im Trennelement fließt. Im Falle von Testkartuschen zur Verwendung im Plättchenfunktionsassay aktivieren Reagenzien auf dem Trennelement die Bildung eines Thrombocytenpfropfs, der die Öffnung verstopft, so dass die Blutprobe nicht mehr durch das Kapillarrohr fließen kann. Die zum Unterbrechen des Blutflusses erforderliche Zeit wird mit der Zeit verglichen, die zum Unterbrechen des Blutflusses erforderlich ist, wenn die Plättchenfunktion des Blutes normal ist. Die Verschlusszeit für normales Blut wird durch Analyse bzw. Testen von normalem Blut ermittelt.

Die bei den bekannten Vorrichtungen eingesetzten porösen Trennelemente in den Testkartuschen eignen sich für Vollblut- und Blutplasmakoagulationsassays, die den Prothrombinzeittests und den partiellen Thromboplastinzeittests zur Bewertung von Gerinnungsfunktionen ähnlich sind. Die Pfropfbildung wird durch Kontakt des Blutes mit geeigneten Aktivatoren für exogene bzw. endogene Aktivierung eingeleitet. Die Aktivatoren sind in die porösen Trennelemente eingebaut. Die zum Stoppen des Blutflusses erforderliche Zeit wird beispielsweise mit der Prothrombinzeit oder der partiellen Thromboplastinzeit der zu untersuchenden Personen korreliert.

Die Konzentration des oder der Mittel in den porösen Trennelemente wird so gewählt, dass sich eine Verschlusszeit ergibt, die einen Unterschied zwischen normalen und anomalen Gerinnungsparametern anzeigt. Beim Plättchenfunktionstest ist Adenosin-5'-diphosphat (ADP) ein bevorzugtes Reagenz für den Einbau in die porösen Trennelemente. Die Verschlusszeit hängt bei einer normalen Blutprobe zum Teil von der Konzentration der biologisch aktiven Substanz ab, die in die Membran eingebaut ist. Die Konzentration dieser Substanz wird so gewählt, dass eine gute Unterscheidung zwischen normalem und anomalem Koagulationsparameter erhalten wird. Die Reagenzkonzentration kann unter Berücksichtigung der gewünschten Empfindlichkeit des Assays optimiert werden. Es ist wünschenswert, dass die Konzentration an ADP ausreichend ist, um eine geringfügige Thrombocytendysfunktion nachweisen zu können, aber nicht so niedrig ist, dass variable Ergebnisse erhalten werden.

Bei den bekannten Vorrichtungen für die Blutplättchendiagnostik stellt sich das Problem die technischen Funktionsabläufe vor Beginn eines Assays zu überprüfen bzw. zu kontrollieren. Diese Funktionskontrolle der Vorrichtungen unterscheidet sich völlig von dem Selbsttest der Vorrichtungen, bei dem beispielsweise Betriebsspannung, Stromaufnahme, Betriebstemperatur, Aufheizzeit für die Probe und dergleichen überprüft werden.

Aufgabe der Erfindung ist es, die technischen Funktionsabläufe einer Vorrichtung für die Blutplättchendiagnostik zu überprüfen.

Diese Aufgabe wird durch die in den Ansprüchen beschriebenen Gegenstände und Verfahren, insbesondere durch eine Vorrichtung der eingangs beschriebenen Art in der Weise gelöst, dass' ein Trennelement ein Flüssigkeitsvolumen und ein darüber befindliches Luftpolster in der Haltekammer luftdicht abschließt, dass eine Messzelle in den oberen Teil der Testkammer einsetzbar ist und dass ein Kapillarrohr die Messzelle mit dem Flüssigkeitsvolumen verbindet.

In Ausgestaltung der Vorrichtung ist die Haltekammer von einem oberen Verschluss luftdicht abschließbar. Die Messzelle sitzt zweckmäßigerweise luftdicht auf einem umlaufenden Sockel auf, der an der Innenwand der Testkammer angebracht ist. An die Messzelle ist abdichtend eine Saugvorrichtung anschließbar, die einen Unterdruck in der Messzelle erzeugt.

In einer bevorzugten Ausgestaltung der Erfindung enthält die Testflüssigkeit ein Gemisch aus Glycerin und Wasser. Aber auch andere Flüssigkeiten oder deren Gemische werden erfindurigsgemäß als Testflüssigkeit eingesetzt, wobei diese je nach speziellem Anwendungszweck im Vergleich zu der Viskosität der zu testenden Probe, z. B. Vollblut, plättchenreiches Blutplasma, Blutplasma, eine vergleichbare, d. h. im Rahmen der normalen Schwankungsbreite identische, niedrigere oder höhere Viskosität aufweisen. Als Testflüssigkeit geeignete Flüssigkeiten sind z. B. Wasser, Glycerin, Öle, Polyethylenglycol und deren Gemische. Die Testflüssigkeit kann auch noch weitere Komponenten enthalten, die z. B. die Stabilität und/oder die Haltbarkeit der Testflüssigkeit erhöhen oder deren Viskosität verändern können, z. B. Puffersubstanzen, Salze, antimikrobiell wirkende Substanzen, Nukleinsäureketten, Kohlehydratketten, Proteine etc. Die Viskosität der zu testenden Probe und der Testflüssigkeit kann z. B. mit handelsüblichen Viskosimetern bestimmt werden.

Ein besonders bevorzugtes erfindungsgemäßes Verfahren zur Funktionskontrolle einer Vorrichtung für die Untersuchung der Blutplättchenfunküon umfasst folgende Schritte:
a) Bereitstellen eines Flüssigkeitsvolumens einer Testflüssigkeit in einer Haltekammer;
b) luftdichtes Abschließen des Flüssigkeitsvolumens und eines darüber befindlichen Luftpolsters mit einem Trennelement;
c) luftdichtes Abschließen der Testkammer mittels eines Dichtungselements und Einbringen einer Messzelle in die Testkammer;
d) Bewegen eines mit einer Messzelle verbundenen Kapillarrohrs innerhalb der Testkammer in Richtung auf das Trennelement zu und Durchstoßen des Trennelements mit dem Kapillarrohr, so dass dieses mit der Testflüssigkeit in Kontakt gelangt bzw. in die Testflüssigkeit eintaucht;
e) Erzeugen eines ausreichenden Unterdrucks in der Messzelle, so dass Testflüssigkeit durch das Kapillarrohr hindurch in die Messzelle fließt;
f) Messen der Zeit, die benötigt wird, bis der Fluss der Testflüssigkeit in die Messzelle aufhört; und
g) Korrelieren der im Schritt (f) gemessenen Zeit mit einem vorbestimmten Referenzwert.

Der vorbestimmte Referenzwert im Schritt g) ist beispielsweise die Zeit für den Blutfluss in einem vorbestimmten Normalbereich der Vorrichtung für die Untersuchung der Blutplättchenfunktion.

Die weitere Ausgestaltung des Verfahrens ergibt sich aus den Verfahrensmaßnahmen der Ansprüche 17 bis 23.

Die Erfindung ermöglicht es, einen Test zur Bestimmung der Verschlusszeit einer normalen Vollblut- oder Plasmaprobe unter Vorgabe einer Anfangsfließgeschwindigkeit und eines angesaugten Gesamtvolumens der Vollblutprobe mit Hilfe einer Testflüssigkeit zu simulieren. Da die zu erwartende Verschlusszeit bekannt ist, zeigt jede größere Abweichung der Verschlusszeit der Testflüssigkeit an, dass die Einsatzbereitschaft der Vorrichtung für die gewünschte Diagnostik, z. B. Blutplättchendiagnostik, nicht gegeben ist. So kann beispielsweise ein Leck zwischen Saugvorrichtung und Messzelle der Vorrichtung zu längeren Verschlusszeiten der Restflüssigkeit führen.

Das Messprinzip für die Verschlusszeit der Testflüssigkeit besteht darin, dass die Zeit von Fließbeginn der Testflüssigkeit an bis zum Fließstopp, verursacht durch den Druckausgleich zwischen dem Unterdruck in der Haltekammer und dem Saugdruck in der Messzelle, in Zeiteinheiten wie z. B. in Sekunden gemessen wird.

Die Reproduzierbarkeit der Verschlusszeit ist sehr gut, wobei die Abweichungen in den Parametern angesaugtes Gesamtvolumen der Testflüssigkeit und Anfangsfließgeschwindigkeit mit 1 bis 2 % sehr gering ausfallen.

Spezielle Ausführungsformen der Erfindung werden im Folgenden anhand von zeichnerisch dargestellten Ausführungsbeispielen näher erläutert. Es zeigen:
Figur 1 in Explosionsdarstellung eine Kartusche zur Funktionskontrolle einer Vorrichtung für die Blutplättchendiagnostik nach der Erfindung;
Figur 2 eine Schnittdarstellung einer ersten Ausführungsform der Kartusche entlang der Linie I-I der Figur 1 in Kontakt mit einem Sauganschluss einer Saugvorrichtung;
Figur 3 eine Schnittdarstellung einer zweiten Ausführungsform der Kartusche entlang der Linie I-I der Figur 1 in Kontakt mit einem Sauganschluss einer Saugvorrichtung.
Figuren 4a Schnittdarstellungen einer dritten und vierten Ausführungsform der Kartusche und 4b entlang der Linie I-I der Figur 1.

Die erfindungsgemäße Kartusche für die Funktionskontrolle wird anhand von Ausführungsformen erläutert, die in Vorrichtungen für die Blutplättchendiagnostik eingesetzt werden. Die äußere Gestalt und die Abmessungen des Gehäuses einer solchen Kartusche stimmen daher mit J den Testkartuschen überein, wie sie in den europäischen Patent EP 0 716 744 B1 und 0 716 745 B1 gezeigt und beschrieben sind.

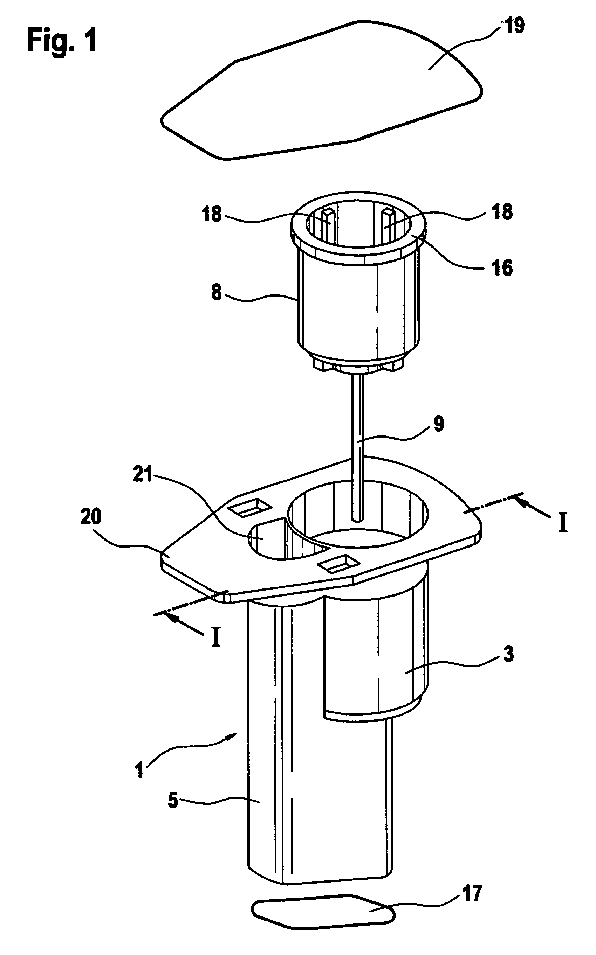

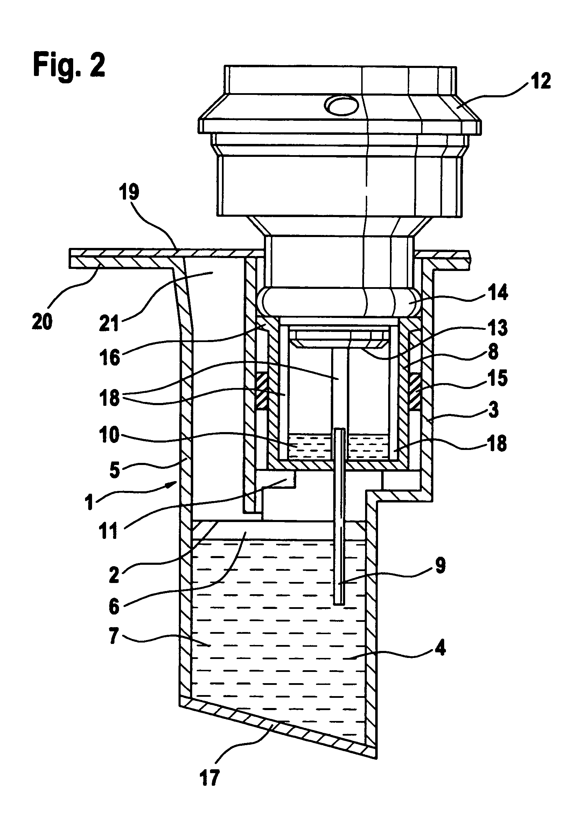

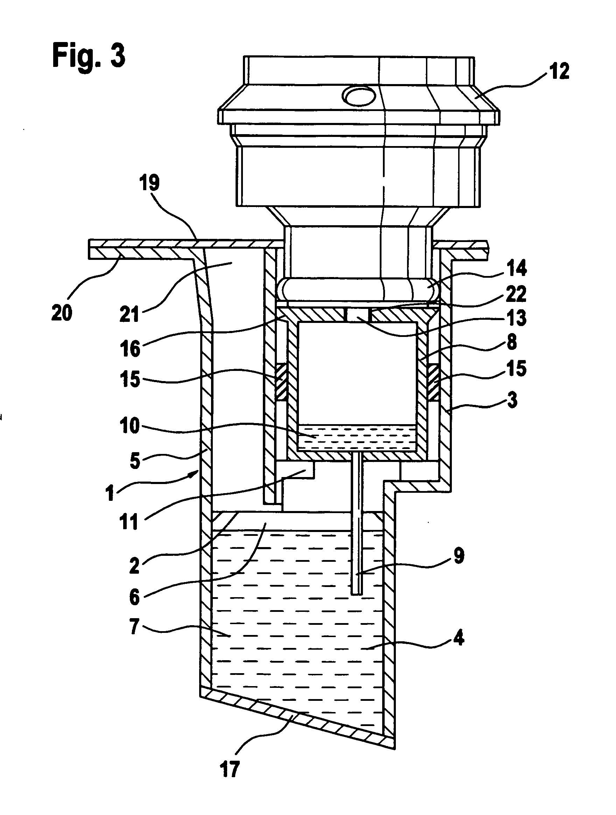

Figur 1 zeigt in Explosionsdarstellung eine Ausführungsform der Kartusche, die ein Gehäuse 1 aufweist, das eine Haltekammer 5 und eine seitlich an der Haltekammer angebrachte Testkammer 3 umschließt. Auf der Oberseite des Gehäuses 1 befindet sich ein umlaufender Flansch 20, der über die Haltekammer 5 und die Testkammer 3 vorspringt. Die Geometrie des Gehäuses 1 ist so gewählt, dass die Wahrscheinlichkeit des Einschlusses einer Luftblase in der Kartusche minimiert wird. Hierzu dient u. a. ein geneigter Boden 17 (s. Figuren 2 und 3) der Haltekammer 5, um den Lufteinschluss klein zu halten, wenn Testflüssigkeit in die Haltekammer 5 durch eine Öffnung 21 eingefüllt wird. Durch die Geometrie des Gehäuses 1 wird ein möglichst großer Oberflächenkontakt der Testflüssigkeit mit der erwärmten Gehäusewand erreicht, während gleichzeitig die Oberfläche der Testflüssigkeit; die der Luft ausgesetzt ist, minimiert wird. In die Testkammer 3 ist eine Messzelle 8 einsetzbar, an der ein Kapillarrohr 9 befestigt ist. Im Inneren der Messzelle 8 sind Sperrrippen 18 zum Positionieren eines Sauganschlusses 13 einer Saugvorrichtung 12 (s. Figuren 2 und 3) angebracht. Insgesamt sind vier derartige Sperrrippen 18 vorgesehen, von denen in Fig. 1 zwei und in Fig. 2 drei gezeigt sind. Nachdem die Messzelle 8 in die Testkammer 3 eingesetzt ist und die Testflüssigkeit durch die Öffnung 21 eingefüllt wurde, wird das Gehäuse 1 mit einem abnehmbaren oberen Verschluss 19 abgeschlossen, der mit dem Flansch 20 abschließt. Der obere Verschluss 19 lässt sich von dem Gehäuse 1 abziehen und vollständig von dem Flansch 20 entfernen.

Die Messzelle 8 weist einen umlaufenden oberen Rand 16 auf.

Die Figuren 2 und 3 zeigen Schnittdarstellungen entlang der Linie I-I von Fig. 1.

Die Schnittansicht der Figur 2 zeigt eine erste Ausführungsform der Kartusche, bei der das Kapillarrohr 9 in das Innere der Messzelle 8 hineinragt. Die Kartusche enthält in der Haltekammer 5 ein Flüssigkeitsvolumen 7. Das Flüssigkeitsvolumen 7 wird durch die eingefüllte Testflüssigkeit 4 aufgebaut. Sowie dies geschehen ist, wird die Haltekammer 5 durch ein Trennelement 2 abgeschlossen und zwar in einer Höhe, dass sich oberhalb des Pegels der Testflüssigkeit 4 ein Luftpolster 6 befindet.

In Figur 2 ist die Konfiguration zwischen der Saugvorrichtung 12 und der Kartusche gezeigt, nachdem der Sauganschluss 13 der Saugvorrichtung 12 mit der Messzelle 8 in Kontakt getreten ist und diese so weit nach unten in der Testkammer gedrückt hat, dass die Messzelle 8 auf einem umlaufenden Sockel 11 aufsitzt, der an der Innenwand der Testkammer 3 angebracht ist. Die Messzelle 8 ist zylindrisch ausgebildet und hat einen Durchmesser kleiner als der Innendurchmesser der Testkammer 3. Der obere leicht ausgewölbte Rand 16 der Messzelle 8 liegt an der Innenwand der Testkammer 3 an. Zwischen der Außenseite der Messzelle 8 und der Innenwand der Testkammer 3 ist ein Dichtungselement 15 angebracht, das die Testkammer luftdicht gegen die Außenatmosphäre abschließt. Bei der Bewegung der Messzelle 8 nach unten durchstößt das Kapillarrohr 9 das Trennelement 2 und gelangt in Kontakt mit der Testflüssigkeit 4 im Flüssigkeitsvolumen 7 bzw. taucht in diese Testflüssigkeit 4 ein. In dieser Stellung der Messzelle 8 wird durch die Saugvorrichtung 12 ein Unterdruck in der Messzelle 8 erzeugt. Der Sauganschluss 13 ist an seiner Außenseite mit einem O-Ring 14 ausgestattet, der auf dem Rand 16 der Messzelle 8 aufliegt und zur Abdichtung der Messzelle gegenüber der Außenatmosphäre beiträgt. Durch den angelegten Unterdruck an die Messzelle 8 strömt Testflüssigkeit 4 aus dem Flüssigkeitsvolumen 7 durch das Kapillarrohr 9 in die Messzelle 8 ein, und es bildet sich ein Flüssigkeitsvolumen 10 mit steigendem Pegel aus. Durch das Einströmen von Testflüssigkeit 4 in die Messzelle 8 nimmt das Flüssigkeitsvolumen 7 ab und das Luftpolster 6 dehnt sich aus, wodurch ein Unterdruck im Luftraum zwischen dem Trennelement 2 und dem Flüssigkeitsvolumen 7 entsteht. Sobald dieser Unterdruck gleich groß wie der von der Saugvorrichtung 12 hervorgerufene Unterdruck in der Messzelle 8 ist, stellt sich ein Gleichgewicht ein und es strömt ab diesem Zeitpunkt keine Testflüssigkeit 4 mehr in die Messzelle 8 ein. Die Zeitspanne vom Fließbeginn der Testflüssigkeit 4, d. h. ab der Erzeugung eines Unterdrucks von beispeilsweise -40 mbar in der Messzelle bis zum Aufhören des Flusses der Testflüssigkeit 4, wird in Sekunden gemessen und als Verschlusszeit bezeichnet. Das der Verschlusszeit zugrunde liegende Messprinzip beruht somit auf einem Gleichgewicht des Unterdrucks im Luftpolster oberhalb des Flüssigkeitsvolumens 7 mit dem Unterdruck in der Messzelle 8.

Die Testflüssigkeit 4 enthält beispielsweise ein Gemisch aus Glycerin und Wasser und ihre Viskosität wird so eingestellt, dass sie der Viskosität von normalem Blut entspricht. Dazu beträgt das Verhältnis Glycerin zu Wasser 30:70 bis 40:60, bezogen auf das Gesamtgewicht der Mischung aus Glycerin und Wasser. Insbesondere beträgt das Verhältnis Glycerin zu Wasser 35:65 Gewichtsprozent, jeweils bezogen auf das Gesamtgewicht der Mischung aus Glycerin und Wasser.

Die anfängliche Fließgeschwindigkeit der Testflüssigkeit 4 wird durch Veränderung der Länge und des Innendurchmessers des Kapillarrohrs 9 gesteuert. Der Innendurchmesser des Kapillarrohrs 9 liegt im Bereich von 100 bis 220 µm, insbesondere beträgt er 150 bis 210 µm. Die Länge des Kapillarrohrs 9 liegt im Bereich von 15 bis 30 mm. In einer bevorzugten Ausführungsform. beträgt der Innendurchmesser des Kapillarrohrs 200 ± 10 µm und die Länge des Kapillarrohrs 30 mm. Die anfängliche Fließgeschwindigkeit (IF = Initial Flow Speed) beträgt 150 bis 200 µl/min mit einer Toleranz von etwa ± 2,5 bis 3 %. Das Gesamtvolumen an Testflüssigkeit 4 beträgt 300 bis 400 µl, mit einer Toleranz von ± 5 bis 7 %. Mit der voranstehend angegebenen Konfiguration des Kapillarohrs 9 und den Bedingungen für die anfängliche Fließgeschwindigkeit und das Gesamtvolumen der Testflüssigkeit 4 ergibt sich eine Verschlusszeit von etwa 120 bis 180 sec, mit einer Toleranz von ± 5 sec. Die Viskosität, das Gesamtvolumen und die anfängliche. Fließgeschwindigkeit der Testflüssigkeit 4 sowie der Unterdruck in der Messzelle 8 bestimmen die Verschlusszeit der Testflüssigkeit. Wenn es für einige Assays erforderlich ist die Verschlusszeit zu verlängern oder zu verkürzen, so kann die Viskosität der Testflüssigkeit 4 durch Änderung des Verhältnisses zwischen Glycerin und Wasser erhöht oder erniedrigt werden. Falls das Kapillarrohr 9 verkürzt wird, kann auch der Innendurchmesser des Kapillarrohrs verringert werden, um die anfängliche Fließgeschwindigkeit beizubehalten. Bei einer Verkürzung des Kapillarrohrs bei gleichbleibendem Innendurchmesser ist das Gesamtvolumen der Testflüssigkeit zu verringern, um die Fließgeschwindigkeit konstant halten zu können. Je größer das Luftvolumen, d..h. das anfängliche Luftpolster 6 über dem Flüssigkeitsvolumen 7 ist, desto länger ist die Verschlusszeit der Testflüssigkeit. Die Größe der anfänglichen Fließgeschwindigkeit, des Gesamtvolumens an Testflüssigkeit, des Luftvolumens bzw. des Luftpolsters 6 und die Viskosität der Testflüssigkeit werden so gewählt, dass die standardisierte Verschlusszeit von 120 bis 180 sec eingehalten wird, die mit der Verschlusszeit von normalem Blut übereinstimmt. Wenn sich bei einer Messung herausstellt, dass die Verschlusszeit der Testflüssigkeit von der standardisierten Verschlusszeit abweicht, kann davon ausgegangen werden, dass die überprüfte Vorrichtung für die Blutplättchendiagnostik nicht einwandfrei funktioniert. Das Material für das Kapillarrohr 9 ist bevorzugt Edelstahl, bei dem die vorgegebene enge Toleranz für den Innendurchmesser einer relativ glatten Innenfläche eingehalten werden kann. Die Messzelle 8 besteht zweckmäßigerweise aus einem Kunststoff wie Polypropylen oder Polyethylenterephthalat.

In Figur 3 ist eine weitere Ausführungsform der Kartusche im Schnitt dargestellt. Diese Ausführungsform unterscheidet sich von der in Figur 2 gezeigten Ausführungsform nur dadurch, dass das Kapillarrohr 9 mit der Messzelle 8 integriert ist, in der Weise, dass das Kapillarrohr integral mit der Unterseite der Messzelle 8 verbunden ist, ohne in die Messzelle hineinzuragen. Die Oberseite der Messzelle 8 ist bis auf eine kleine Öffnung 22 geschlossen. Diese Öffnung 22 ist mit einem Sauganschluss der Saugvorrichtung 12 verbunden. In der in Figur 3 gezeigten Position hat das Kapillarrohr 9 gleichfalls das Trennelement 2 durchstoßen und steht in Kontakt mit der Testflüssigkeit 4 des Flüssigkeitsvolumens 7. Die Saugvorrichtung 12, die über den O-Ring 14 dichtend auf der Messzelle 8 aufliegt, hat die Messzelle 8 in der Testkammer 3 so weit nach unten geschoben, dass die Messzelle auf dem Sockel 11 aufsitzt. Bei dieser Ausführungsform der Messzelle 8 können die Sperrrippen entfallen, da die Messzelle allseitig bis auf die kleine Öffnung 22 geschlossen ist. Sobald die Saugvorrichtung 12 innerhalb der Messzelle 8 einen Unterdruck erzeugt, steigt die Testflüssigkeit 4 durch das Kapillarrohr 9 in die Messzelle hoch und bildet dort so lange ein ansteigendes Flüssigkeitsvolumen 10 aus, bis der Unterdruck im Luftpolster 6 oberhalb des Flüssigkeitsvolumens 7 gleich dem angelegten Unterdruck in der Messzelle 8 ist.
Die zur einmaligen Verwendung vorgesehene Kartusche wird zusammen mit der Testflüssigkeit 4 innerhalb der Messzelle 8 am Ende des Testes verworfen. Die in den Figuren 2 und 3 gezeigte Kartusche zur einmaligen Verwendung wird für den Test durch folgende Schritte vorbereitet. Zunächst wird die Testflüssigkeit 4 in die Haltekammer 5 eingefüllt und anschließend die Haltekammer 5 mit dem Trennelement 2 luftdicht abgeschlossen. Es befindet sich zwischen dem Trennelement 2 und dem Flüssigkeitsvolumen 7 ein Luftpolster 6. Als nächstes wird eine Messzelle 8 in die Testkammer 3 eingebracht und die Testkammer mittels eines Dichtungselements 15, das an der Innenwand des Gehäuses 1 der Kartusche und einer Zwischenwand zwischen der Testkammer 3 und der Haltekammer 5 anliegt, luftdicht abgeschlossen. Danach wird die Oberseite des Gehäuses 1 mit einem Verschluss 19 luftdicht verschlossen. Die Messzelle 8 befindet sich in einer Position nächst dem Verschluss 19, wobei das mit der Messzelle 8 verbundene Kapillarrohr 9 mit seinem unteren Ende oberhalb des Trennelements 2 angeordnet ist, d. h. das Trennelement 2 von dem Kapillarrohr 9 nicht durchstoßen ist. Als nächstes wird dann vor dem Einsetzen der Kartusche in die Vorrichtung für die Blutplättchendiagnostik der Verschluss 19 über der Testkammer 3 entfernt. Soweit der Verschluss 19 die Haltekammer 5 abdeckt, bleibt er erhalten. Im nächsten Schritt wird das mit der Messzelle 8 verbundene Kapillarrohr 9 innerhalb der Testkammer 3 in Richtung auf das Trennelement 2 bewegt, verursacht durch den von der Saugvorrichtung 12 ausgeübten mechanischen Druck auf die Messzelle 8, wodurch diese nach unten bis zum Aufsetzen auf den Sockel 11 geschoben wird. Bei dieser Bewegung durchstößt das Kapillarrohr 9 das Trennelement und gelangt mit der Testflüssigkeit 4 des Flüssigkeitsvolumens 7 in Kontakt bzw. taucht in die Testflüssigkeit ein. Durch die Saugvorrichtung 12 wird ein ausreichender Unterdruck in der Messzelle erzeugt, so dass Testflüssigkeit durch das Kapillarrohr 9 in die Messzelle fließt und dort ein Flüssigkeitsvolumen 10 aufgebaut wird. Es wird die Zeit gemessen, die benötigt wird, bis der Fluss der Testflüssigkeit in die Messzelle aufhört. Den Beginn der Zeitmessung markiert das Anlegen des Unterdrucks an die Messzelle. Die so erhaltene Verschlusszeit wird mit der Zeit für den Blutfluss in einem vorbestimmten Normalbereich der Vorrichtung für die Blutplättchendiagnostik korreliert.

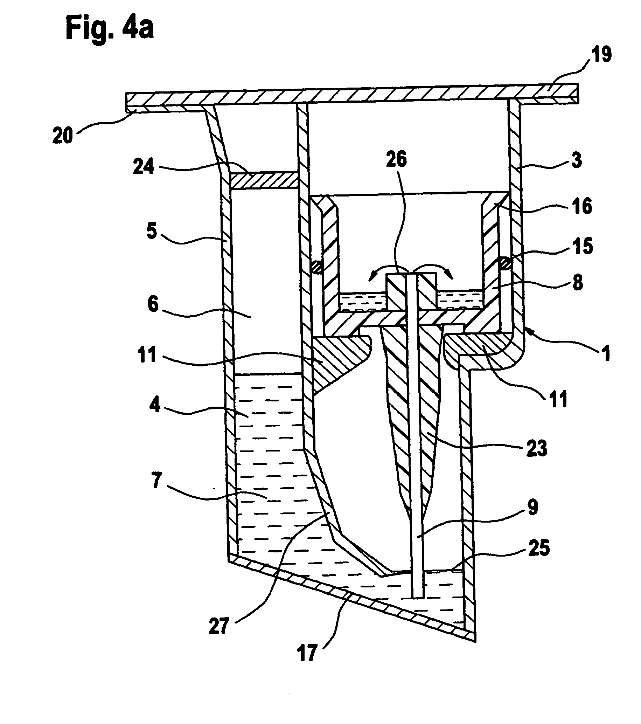

Figur 4a zeigt eine dritte Ausführungsform der Kartusche, die ähnlich der ersten Ausführungsform gemäß Figur 2 ausgestaltet ist.

Das Dichtungselement 15 ist bei dieser Ausführungsform als O-Ring ausgebildet. Das Kapillarrohr ist innerhalb der Haltekammer 5 von einer Ummantelung 23 umgeben und soweit das Kapillarrohr 9 in die Messzelle 8 hineinragt, wird es von einer weiteren Ummantelung 26 umhüllt. Obgleich dies in den Ausführungsformen gemäß den Figuren 2 und 3 nicht gezeigt ist, ist es selbstverständlich, dass auch bei der ersten und zweiten Ausführungsform das Kapillarrohr 9 jeweils von einer Ummantelung 23 umgeben ist oder sein kann und dass die erste Ausführungsform noch zusätzlich eine Ummantelung 26 aufweist oder aufweisen kann. Das Kapillarrohr 9 durchsetzt ein Trennelement 25.

Eine Wand 27 zwischen der Haltekammer 5 und der Testkammer 3 reicht weit nach unten bis nahe an den geneigten Boden 17 und verläuft im unteren Abschnitt geknickt oder gekrümmt. Das Trennelement 25 befindet sich zwischen der Wand und einer Wand des Gehäuses 1 und verschließt die Haltekammer 5 gegenüber der Testkammer 3. Die Testflüssigkeit nimmt ein Flüssigkeitsvolumen 7 innerhalb der Haltekammer 5 ein, wobei das Flüssigkeitsvolumen etwa das halbe Volumen der Haltekammer 5 ausfüllt. Im oberen Teil des Haltekammer 5 befindet sich ein weiteres Trennelement 24, das nach dem Einfüllen der Testflüssigkeit 4 in die Haltekammer 5 mit Abstand zu dem Flansch 20 eingefügt wird. Zwischen diesem Trennelement 24 und dem Pegel der Testflüssigkeit 4 ist das Luftpolster 6 eingeschlossen. Die übrigen Teile der Kartusche entsprechend weitgehend der ersten Ausführungsform gemäß Figur 2 und sind mit den gleichen Bezugszeichen wie in Figur 2 belegt, so dass sie nicht nochmals beschrieben werden.

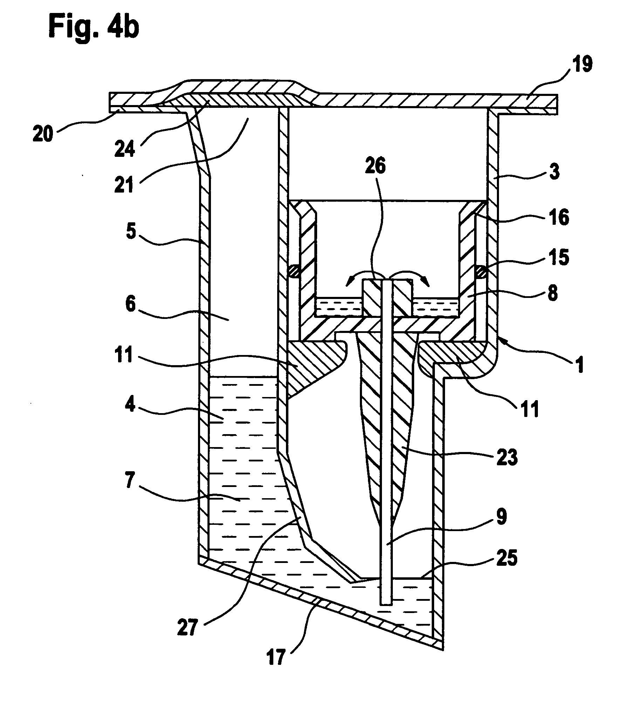

Die vierte Ausführungsform nach Figur 4b ist ähnlich der dritten Ausführungsform nach Figur 4a aufgebaut, so dass nur die unterschiedlichen Merkmale zu der dritten Ausführungsform im Folgenden erläutert werden. Bei der dargestellten vierten Ausführungsform wird die Öffnung 21 der Haltekammer 5 nach dem Einfüllen der Testflüssigkeit mit dem Trennelement 24 verschlossen, das auf dem Flansch 20 aufliegt. Der obere Verschluss 19 der Kartusche überwölbt das Trennelement 24 und schließt mit dem Flansch 20 ab. Nach dem Abziehen des Verschlusses 19 von dem Flansch 20 bleibt die Haltekammer 5 weiterhin durch das Trennelement 24 luftdicht verschlossen. Der Verschluss 19 besteht beispielsweise aus einer selbstklebenden Folie. Das Luftpolster 6 ist bei der vierten Ausführungsform größer als bei der dritten Ausführungsform, da das Trennelement 24 die Öffnung 21 der Haltekammer bündig abschließt und nicht innerhalb der Haltekammer, wie bei der dritten Ausführungsform, angebracht ist.

Das Kapillarrohr 9 durchdringt das Trennelement 25.

Die Konfiguration zwischen der Saugvorrichtung und der Kartusche ist für die dritte und vierte Ausführungsform die gleiche wie sie anhand der ersten Ausführungsform beschrieben ist. Aus Gründen der besseren Übersicht ist in den Figuren 4a und 4b die Saugvorrichtung nicht dargestellt, jedoch gilt; dass der von der Saugvorrichtung ausgeübte Unterdruck die Testflüssigkeit durch das Kapillarrohr 9 in die Messzelle 8 einströmen lässt und dort das Flüssigkeitsvolumen 10 so weit aufbaut, bis der Unterdruck im Luftpolster 6 oberhalb des Flüssigkeitsvolumens 7 gleich dem angelegten Unterdruck in der Messzelle 8 ist.

Durch längere oder kürzere Verschlusszeiten im Vergleich mit der standardisierten Verschlusszeit für normales Blut oder.anderen zu testenden Proben und höhere oder niedrigere anfängliche Fließgeschwindigkeiten der Testflüssigkeit können neben normalen auch anomale Blutzustände simuliert und damit die Funktionskontrolle von Vorrichtungen für die Blutplättchendiagnostik in Bezug auf anomale Blutzusammensetzungen durchgeführt werden. Dies kann durch die Auswahl der entsprechend geeigneten Testflüssigkeit, insbesondere über deren Viskosität im Vergleich mit der Viskosität der zu testenden Probe, in den erfindungsgemäßen Kartuschen sowie anderen erfindungsgemäßen Ausgestaltungsformen erreicht werden.

## Patentansprüche

1. Kartusche zur Funktionskontrolle einer Vorrichtung für die Untersuchung der Blutplättchenfunktion, mit einem Gehäuse (1), das eine Testkammer (3) und eine Haltekammer (5) umschließt, **dadurch gekennzeichnet, dass** ein Trennelement (2; 25) das Flüssigkeitsvolumen (7) einer Testflüssigkeit (4) und ein darüber befindliches Luftpolster (6) in der Haltekammer (5) luftdicht abschließt, dass eine Messzelle (8) in den oberen Teil der Testkammer (3) einsetzbar ist und dass ein Kapillarrohr (9) die Messzelle mit dem Flüssigkeitsvolumen (7) verbindet.

2. Kartusche nach Anspruch 1, **dadurch gekennzeichnet, dass** die Haltekammer (5) von einem oberen Verschluss (19) luftdicht abschließbar ist.

3. Kartusche nach Anspruch 1 oder 2, **dadurch gekennzeichnet, dass** die Messzelle (8) auf einem umlaufenden Sockel (11) aufsitzt, der an der Innenwand der Testkammer (3) angebracht ist.

4. Kartusche nach einem oder mehreren der Ansprüche 1 bis 3, **dadurch gekennzeichnet, dass** die Messzelle (8) zylindrisch ist und einen Durchmesser kleiner als der Innendurchmesser der Testkammer (3) aufweist.

5. Kartusche nach einem oder mehreren der Ansprüche 1 bis 4, **dadurch gekennzeichnet, dass** ein Dichtungselement (15) zwischen der Außenseite der Messzelle (8) und der Innenwand der Testkammer (3) die Testkammer luftdicht gegen die Außenatmosphäre abschließt.

6. Kartusche nach einem oder mehreren der Ansprüche 1 bis 5, **dadurch gekennzeichnet, dass** an die Messzelle (8) abdichtend eine Saugvorrichtung (12) anschließbar ist, die einen Unterdruck in der Messzelle (8) erzeugt.

7. Kartusche nach Anspruch 6, **dadurch gekennzeichnet, dass** die Saugvorrichtung (12) einen Sauganschluss (13) aufweist, der mit einem O-Ring (14) ausgerüstet ist.

8. Kartusche nach Anspruch 7, **dadurch gekennzeichnet, dass** der O-Ring (14) dichtend auf einem Rand (16) der Messzelle (8) aufliegt.

9. Kartusche nach einem oder mehreren der Ansprüche 1 bis 8, **dadurch gekennzeichnet, dass** das Kapillarrohr (9) mit seinem oberen Ende in die Messzelle (8) hineinragt, das Trennelement (2) durchsetzt und mit seinem unteren Ende in die Testflüssigkeit (4) des Flüssigkeitsvolumens (7) eintaucht.

10. Kartusche nach einem oder mehreren der Ansprüche 1 bis 9, **dadurch gekennzeichnet, dass** die Messzelle (8) bis auf eine kleine Öffnung (22) in ihrer Oberseite und dem mit der Messzelle (8) verbundenen Kapillarrohr (9) allseitig geschlossen ist.

11. Kartusche nach Anspruch 10, **dadurch gekennzeichnet, dass** das Kapillarrohr (9) integral mit der Unterseite der Messzelle (8) verbunden ist, ohne in die Messzelle (8) hineinzuragen.

12. Kartusche nach einem oder mehreren der Ansprüche 1 bis 11, **dadurch gekennzeichnet, dass** die Testflüssigkeit (4) ein Gemisch aus Glycerin und Wasser enthält.

13. Kartusche nach Anspruch 12, **dadurch gekennzeichnet, dass** das Verhältnis Glycerin zu Wasser 30:70 bis 40:60, bevorzugt 35:65 Gewichtsprozent beträgt, bezogen auf das Gesamtgewicht der Mischung aus Glycerin und Wasser.

14. Kartusche nach einem oder mehreren der Ansprüche 1 bis 11, **dadurch gekennzeichnet, dass** die Testflüssigkeit Wasser, Öle, Polyethyenglycol oder Mischungen hiervon enthält.

15. Kartusche nach Anspruch 12 oder 14, **dadurch gekennzeichnet, dass** die Testflüssigkeit (4) Puffersubstanzen, Salze, antimikrobiell wirkende Substanzen, Nukleinsäureketten, Kohlehydratketten, Proteine enthält.

16. Verfahren zur Funktionskontrolle einer Vorrichtung für die Untersuchung der Blutplättchenfunktion, das folgende Schritte umfasst:
a) Bereitstellen eines Flüssigkeitsvolumens (7) einer Testflüssigkeit (4) in einer Haltekammer (5);
b) luftdichtes Abschließen des Flüssigkeitsvolumens (7) und eines darüberbefindlichen Luftpolsters (6) mit einem Trennelement (2; 25);
c) luftdichtes Abschließen der Testkammer (3) mittels eines Dichtungselements (15) und Einbringen einer Messzelle (8) in die Testkammer;
d) Bewegen eines mit einer Messzelle (8) verbundenen Kapillarrohrs (9) innerhalb der Testkammer (3) in Richtung auf das Trennelement (2; 25) zu und Durchstoßen des Trennelements mit dem Kapillarrohr, so dass dieses mit der Testflüssigkeit in Kontakt gelangt bzw. in die Testflüssigkeit eintaucht;
e) Erzeugen eines ausreichenden Unterdrucks in der Messzelle, so dass Testflüssigkeit durch das Kapillarrohr hindurch in die Messzelle fließt;
f) Messen der Zeit, die benötigt wird, bis der Fluss der Testflüssigkeit in die Messzelle aufhört; und
g) Korrelieren der im Schritt (f) gemessenen Zeit mit einem vorbestimmten Referenzwert.

17. Verfahren nach Anspruch 16, **dadurch gekennzeichnet, dass** die Viskosität der Testflüssigkeit gleich der Viskosität von Blut oder Blutplasma im Normalzustand gewählt wird.

18. Verfahren nach Anspruch 16, **dadurch gekennzeichnet, dass** die Viskosität der Testflüssigkeit höher als die Viskosität von Blut oder Blutplasma im Normalzustand gewählt wird, so dass die Zeit gemäß Schritt (f) sich verlängert.

19. Verfahren nach Anspruch 16, **dadurch gekennzeichnet, dass** die Viskosität der Testflüssigkeit niedriger als die Viskosität von Blut oder Blutplasma im Normalzustand gewählt wird, so dass die Zeit gemäß Schritt (f) sich verkürzt.

20. Verfahren nach einem oder mehreren der Ansprüche 16 bis 19, **dadurch gekennzeichnet, dass** das Flüssigkeitsvolumen der Testflüssigkeit gegenüber dem Anfangszustand verringert wird, um die Zeit gemäß Schritt (f) zu verlängern.

21. Verfahren nach einem oder mehreren der Ansprüche 16 bis 20, **dadurch gekennzeichnet, dass** das Kapillarrohr verlängert wird, um die Zeit gemäß Schritt (f) zu verlängern.

22. Verfahren nach einem oder mehreren der Ansprüche 16 bis 21, **dadurch gekennzeichnet, dass** der Innendurchmesser des Kapillarrohrs verringert wird, um die Zeit gemäß Schritt (f) zu verlängern.

23. Verfahren nach Anspruch 16, **dadurch gekennzeichnet, dass** die Viskosität der Testflüssigkeit vergleichbar, niedriger oder höher ist als die Viskosität der zu testenden Probe.

24. Verfahren nach einem der Ansprüche 16 und 23, **dadurch gekennzeichnet, dass** es sich bei der Testflüssigkeit um Wasser, Glycerin, Öle, Polyethylenglycol oder um ein Gemisch derselben handelt, welche ferner bevorzugterweise Puffersubstanzen, Salze, antimikrobiell wirkende Substanzen, Nukleinsäureketten, Kohlehydratketten, Proteine oder Gemische derselben enthält.

## Claims

1. Cartridge for monitoring the function of a device for testing blood platelet function, with a housing (1) which includes a test chamber (3) and a holding chamber (5), **characterized in that** a separating element (2; 25) closes off in an airtight manner the fluid volume (7) of a test fluid (4) and an air cushion (6) situated above the latter in the holding chamber (5), a measurement cell (8) can be fitted into the upper part of the test chamber (3), and a capillary tube (9) connects the measurement cell to the fluid volume (7).

2. Cartridge according to Claim 1, **characterized in that** the holding chamber (5) can be closed off in an airtight manner by an upper closure element (19).

3. Cartridge according to Claim 1 or 2, **characterized in that** the measurement cell (8) sits on a peripheral base (11) arranged on the inner wall of the test chamber (3).

4. Cartridge according to one or more of Claims 1 to 3, **characterized in that** the measurement cell (8) is cylindrical and has a diameter smaller than the internal diameter of the test chamber (3).

5. Cartridge according to one or more of Claims 1 to 4, **characterized in that** a sealing element (15) between the outside of the measurement cell (8) and the inner wall of the test chamber (3) closes the test chamber off in an airtight manner from the outside atmosphere.

6. Cartridge according to one or more of Claims 1 to 5, **characterized in that** a suction device (12) can be connected sealingly to the measurement cell (8) and generates an underpressure in the measurement cell (8).

7. Cartridge according to Claim 6, **characterized in that** the suction device (12) has a suction attachment (13) which is equipped with an O-ring (14).

8. Cartridge according to Claim 7, **characterized in that** the O-ring (14) lies sealingly on an edge (16) of the measurement cell (8).

9. Cartridge according to one or more of Claims 1 to 8, **characterized in that** the capillary tube (9), with its upper end, protrudes into the measurement cell (8), passes through the separating element (2) and, with its lower end, plunges into the test fluid (4) of the fluid volume (7).

10. Cartridge according to one or more of Claims 1 to 9, **characterized in that** the measurement cell (8) is closed all round, except for a small opening (22) in its top face and except for the capillary tube (9) connected to the measurement cell (8).

11. Cartridge according to Claim 10, **characterized in that** the capillary tube (9) is connected integrally to the underside of the measurement cell (8) without protruding into the measurement cell (8).

12. Cartridge according to one or more of Claims 1 to 11, **characterized in that** the test fluid (4) contains a mixture of glycerol and water.

13. Cartridge according to Claim 12, **characterized in that** the ratio of glycerol to water is 30:70 to 40:60, preferably 35:65 percent by weight, based on the total weight of the mixture of glycerol and water.

14. Cartridge according to one or more of Claims 1 to 11, **characterized in that** the test fluid contains water, oils, polyethylene glycol or mixtures thereof.

15. Cartridge according to Claim 12 or 14, **characterized in that** the test fluid (4) contains buffer substances, salts, antimicrobial substances, nucleic acid chains, carbohydrate chains, proteins.

16. Method for monitoring the function of a device for testing blood platelet function, which method comprises the following steps:
(a) making ready a fluid volume (7) of a test fluid (4) in a holding chamber (5);
(b) closing off the fluid volume (7), and an air cushion (6) located above this, in an airtight manner by means of a separating element (2; 25);
(c) closing off the test chamber (3) in an airtight manner by means of a sealing element (15) and introducing a measurement cell (8) into the test chamber;
(d) moving a capillary tube (9), connected to a measurement cell (8), inside the test chamber (3) in the direction of the separating element (2; 25) and piercing the separating element with the capillary tube so that the latter comes into contact with the test fluid or plunges into the test fluid;
(e) generating a sufficient underpressure in the measurement cell so that test fluid flows through the capillary tube into the measurement cell;
(f) measuring the time needed until the flow of the test fluid into the measurement cell stops; and
(g) correlating the time measured in step (f) with a predetermined reference value.

17. Method according to Claim 16, **characterized in that** the viscosity of the test fluid is chosen equal to the viscosity of blood or blood plasma in the normal state.

18. Method according to Claim 16, **characterized in that** the viscosity of the test fluid is chosen greater than the viscosity of blood or blood plasma in the normal state, so that the time according to step (f) is lengthened.

19. Method according to Claim 16, **characterized in that** the viscosity of the test fluid is chosen lower than the viscosity of blood or blood plasma in the normal state, so that the time according to step (f) is shortened.

20. Method according to one or more of Claims 16 to 19, **characterized in that** the fluid volume of the test fluid is reduced compared to the initial state in order to lengthen the time according to step (f).

21. Method according to one or more of Claims 16 to 20, **characterized in that** the capillary tube is lengthened in order to lengthen the time according to step (f).

22. Method according to one or more of Claims 16 to 21, **characterized in that** the internal diameter of the capillary tube is reduced in order to lengthen the time according to step (f).

23. Method according to Claim 16, **characterized in that** the viscosity of the test fluid is comparable to or lower or higher than the viscosity of the sample to be tested.

24. Method according to one of Claims 16 and 23, **characterized in that** the test fluid is water, glycerol, oils, polyethylene glycol or a mixture of these, which furthermore preferably contains buffer substances, salts, antimicrobial substances, nucleic acid chains, carbohydrate chains, proteins or mixtures of these.

## Revendications

1. Cartouche pour le contrôle de la fonctionnalité d'un dispositif d'analyse de la fonctionnalité des plaquettes sanguines, comprenant un boîtier ( 1 ) qui renferme une chambre ( 3 ) de test et une chambre ( 5 ) de maintien, **caractérisé en ce qu'**un élément ( 2; 25 ) de séparation ferme, de manière étanche à l'air, le volume ( 7 ) d'un liquide ( 4 ) de test et un coussin ( 6 ) d'air se trouvant dessus dans la chambre ( 5 ) de maintien, **en ce qu'**une cellule ( 8 ) de mesure peut être insérée dans la partie supérieure de la chambre ( 3 ) de test et **en ce qu'**un tube ( 9 ) capillaire met la cellule de mesure en communication avec le volume ( 7 ) de liquide.

2. Cartouche suivant la revendication 1, **caractérisée en ce que** la chambre ( 5 ) de maintien peut être fermée d'une manière étanche à l'air par une fermeture ( 19 ) supérieure.

3. Cartouche suivant la revendication 1 ou 2, **caractérisée en ce que** la cellule ( 8 ) de mesure est posée sur un socle ( 11 ) faisant le tour, qui est mis sur la paroi intérieure de la chambre ( 3 ) de test.

4. Cartouche suivant l'une ou plusieurs des revendications 1 à 3, **caractérisée en ce que** la cellule ( 8 ) de mesure est cylindrique et a un diamètre plus petit que le diamètre intérieur de la chambre ( 3 ) de test.

5. Cartouche suivant l'une ou plusieurs des revendications 1 à 4, **caractérisée en ce qu'**un élément ( 15 ) d'étanchéité, entre le côté de la cellule ( 8 ) de mesure et la paroi intérieure de la chambre ( 3 ) de test, ferme la chambre de test d'une manière étanche à l'air, vis-à-vis de l'atmosphère extérieure.

6. Cartouche suivant l'une ou plusieurs des revendications 1 à 5, **caractérisée en ce qu'**à la cellule ( 8 ) de mesure peut être raccordée, d'une manière étanche, un dispositif ( 12 ) d'aspiration, qui produit une dépression dans la cellule ( 8 ) de mesure.

7. Cartouche suivant la revendication 6, **caractérisée en ce que** le dispositif ( 12 ) d'aspiration a un raccord ( 13 ) d'aspiration, qui est équipé d'un joint ( 14 ) torique.

8. Cartouche suivant la revendication 7, **caractérisée en ce que** le joint ( 14 ) torique s'applique d'une manière étanche à un bord ( 16 ) de la cellule ( 8 ) de mesure.

9. Cartouche suivant l'une ou plusieurs des revendications 1 à 8, **caractérisée en ce que** le tube ( 9 ) capillaire pénètre, par son extrémité supérieure, dans la cellule ( 8 ) de mesure, traverse l'élément ( 2 ) de séparation et plonge par son extrémité inférieure dans le liquide ( 4 ) de test du volume ( 7 ) de liquide.

10. cartouche suivant l'une ou plusieurs des revendications 1 à 9, **caractérisée en ce que** la cellule ( 8 ) de mesure est fermée de tous côtés, à l'exception d'une petite ouverture ( 12 ) dans son côté supérieur et du tube ( 9 ) capillaire relié à la cellule ( 8 ) de mesure.

11. Cartouche suivant la revendication 10, **caractérisée en ce que** le tube ( 9 ) capillaire est relié d'une seule pièce avec le côté inférieur de la cellule ( 8 ) de mesure, sans pénétrer dans la cellule ( 8 ) de mesure.

12. Cartouche suivant l'une ou plusieurs des revendications 1 à 11, **caractérisée en ce que** le liquide ( 4 ) de test contient un mélange de glycérine et d'eau.

13. Cartouche suivant la revendication 12, **caractérisée en ce que** le rapport de la glycérine à l'eau va de 30:70 à 40:60, en étant de préférence de 35:65 pourcent en poids, rapporté au poids total du mélange de glycérine et d'eau.

14. Cartouche suivant l'une ou plusieurs des revendications 1 à 11, **caractérisée en ce que** le liquide de test contient de l'eau, des huiles, du polyéthylèneglycol ou leurs mélanges.

15. Cartouche suivant la revendication 12 ou 14, **caractérisée en ce que** le liquide ( 4 ) de test contient des substances tampon, des sels, des substances antimicrobiennes, des chaînes d'acides nucléiques, des chaînes d'hydrates de carbone, des protéines.

16. Procédé de contrôle de la fonctionnalité d'un dispositif d'analyse de la fonctionnalité des plaquettes sanguines, comprenant les stades suivantes :
a) on se procure un volume ( 7 ) d'un liquide ( 4 ) de test dans une chambre ( 5 ) de maintien ;
b) on ferme, d'une manière étanche à l'air, le volume ( 7 ) de liquide et un coussin ( 6 ) d'air se trouvant au-dessus, par un élément ( 2 ; 25 ) de séparation ;
c) on ferme, d'une manière étanche à l'air, la chambre ( 3 ) de test, au moyen d'un élément ( 15 ) d'étanchéité et on met une cellule ( 8 ) de mesure dans la chambre de test,
d) on déplace un tube ( 9 ) capillaire relié à une cellule ( 8 ) de mesure, à l'intérieur de la chambre ( 3 ) de test en direction de l'élément ( 2 ; 25 ) de séparation et on perce l'élément de séparation par le tube capillaire, de manière à ce que celui-ci vienne en contact avec le liquide de test ou plonge dans le liquide de test ;
e) on produit une dépression suffisante dans la cellule de mesure, de sorte que du liquide de test s'écoule dans la cellule de mesure en passant par le tube capillaire ;
f) on mesure le temps qui est nécessaire jusqu'à ce que le flux du liquide de test dans la cellule de mesure cesse ; et
g) on corrèle le temps mesuré au stade ( f ) à une valeur de référence déterminée à l'avance.

17. Procédé suivant la revendication 16, **caractérisé en ce que** l'on choisit la viscosité du liquide de test égale à la viscosité du sang ou du plasma sanguin à l'état normal.

18. Procédé suivant la revendication 16, **caractérisé en ce que** l'on choisit la viscosité du liquide de test plus grande que la viscosité du sang ou du plasma sanguin à l'état normal, de manière à allonger le temps suivant le stade ( f ).

19. Procédé suivant la revendication 16, **caractérisé en ce que** l'on choisit la viscosité du liquide de test plus petite que la viscosité du sang ou du plasma sanguin à l'état normal, de manière à écourter le temps suivant le stade ( f ).

20. Procédé suivant l'une ou plusieurs des revendications 16 à 19, **caractérisé en ce que** l'on diminue le volume du liquide de test par rapport à l'état initial, pour allonger le temps suivant le stade ( f ).

21. Procédé suivant l'une ou plusieurs des revendications 16 à 20, **caractérisé en ce que** l'on allonge le tube capillaire, pour allonger le temps suivant le stade ( f ).

22. Procédé suivant l'une ou plusieurs des revendications 16 à 21, **caractérisé en ce que** l'on diminue le diamètre intérieur du tube capillaire, pour allonger le temps suivant le stade ( f ).

23. Procédé suivant la revendication 16, **caractérisé en ce que** la viscosité du liquide de test est comparable, plus petite ou plus grande, que la viscosité de l'échantillon à tester.

24. Procédé suivant l'une des revendications 16 et 23, **caractérisé en ce que** le liquide de test est de l'eau, de la glycérine, des huiles, du polyéthylèneglycol ou l'un de leurs mélanges, et contient en outre, de préférence des substances tampon, des sels, des substances antimicrobiennes, des chaînes d'acides nucléiques, des chaînes d'hydrates de carbone, des protéines ou leurs mélanges.
